(19) Europäisches Patentamt
European Patent Office
Office européen des brevets

(11) Veröffentlichungsnummer: **0 005 232**
**B1**

(12) # EUROPÄISCHE PATENTSCHRIFT

(45) Veröffentlichungstag der Patentschrift: **25.08.82**

(51) Int. Cl.³: **C 07 D 217/22,** C 07 D 401/04, C 07 D 403/04, C 07 D 409/14, A 61 K 31/47

(21) Anmeldenummer: **79101233.9**

(22) Anmeldetag: **24.04.79**

(54) **Isochinolinderivate, Verfahren zu ihrer Herstellung und diese enthaltende pharmazeutische Zubereitungen.**

(30) Priorität: **27.04.78 DE 2818403**

(43) Veröffentlichungstag der Anmeldung: **14.11.79 Patentblatt 79/23**

(45) Bekanntmachung des Hinweises auf die Patenterteilung: **25.08.82 Patentblatt 82/34**

(84) Benannte Vertragsstaaten: **BE CH DE FR GB IT NL SE**

(56) Entgegenhaltungen:
**AU-D-4 983 472**
**DE-A-2 030 875**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Bartmann, Wilhelm, Dr., Am Dachsbau 5, D-6232 Bad Soden am Taunus (DE)**
Erfinder: **Konz, Elmar, Dr., Buchenweg 22, D-6232 Bad Soden am Taunus (DE)**
Erfinder: **Geyer, Harry Maurice, Dr., Feldbergstrasse 74, D-6233 Kelkheim (Taunus) (DE)**

**EP 0 005 232 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Isochinolinderivate, Verfahren zu ihrer Herstellung und diese enthaltende pharmazeutische Zubereitungen

3-Amino-4-phenyl-isochinolinderivate mit Wirkungen auf das Zentralnervensystem werden in der DE-A 2 030 675 beschrieben. Im zweiten Absatz auf Seite 17 wird eine neuromuskuläre Blockierungswirkung, anästhetische Wirkung und analgetische Wirkung und somit eine Brauchbarkeit dieser Verbindungen als ZNS-Depressantien angesprochen. Es ist daher überraschend, daß die Isochinolinderivate gemäß der Erfindung eine entgegengesetzte Wirkung auf das Zentralnervensystem ausüben; sie wirken antidepressiv. Weiterhin sind 3-Piperazinoisochinoline mit starker Hemmwirkung auf die Thrombocytenaggregation in der DE-A 2 503 961 beschrieben.

Es wurden in 3-Stellung basisch substituierte Isochinoline gefunden, die wertvolle pharmakologische, insbesondere psychotrope Eigenschaften besitzen.

Gegenstand der Erfindung sind Isochinoline der allgemeinen Formel I

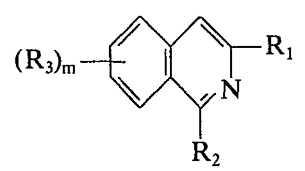

(I)

worin

m eins oder zwei bedeutet,

$R_1$ entweder einen über das Ringstickstoffatom gebundenen Pyrrolidino-, Piperidino-, Hexamethylenimino-, Morpholino-, 4-Hydroxypiperidino-, 4-Carbäthoxypiperidino- oder den 1-Piperazinylrest

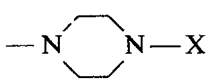

darstellt, worin X Wasserstoff, $C_1-C_4$-Alkyl, $\beta$-Hydroxyäthyl, Methylen-3,4-dioxybenzyl, Phenyl, durch Methoxy, Chlor, Nitro oder Amino substituiertes Phenyl, 3,4,5-Trimethoxybenzoyl, Methylen-3,4-dioxybenzoyl, 2-Furoyl, 2-Thienyl, $C_1-C_3$-Alkoxycarbonyl bedeutet, wobei der $C_1-C_3$-Alkylrest im letzteren durch OH, Methoxy oder Äthoxy substituiert sein kann, oder ein Aminorest der Formel

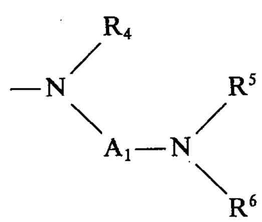

ist, in dem $R_4$ Wasserstoff oder $C_1-C_4$-Alkyl, $A_1$ eine geradkettige oder verzweigte $C_2-C_6$-Alkylengruppe, die durch Hydroxy- oder $C_1-C_4$-Alkoxygruppen substituiert sein kann, $R_6$ und $R_7$ gleich oder verschieden sind und Wasserstoff oder einen geradkettigen oder verzweigten Alkylrest mit 1 bis 6 Kohlenstoffatomen bedeuten oder zusammen mit dem Stickstoffatom einen heterocyclischen Ring mit bis zu 7 Kohlenstoffatomen darstellen,

$R_2$ einen Phenylrest bedeutet, der gegebenenfalls mit Halogen, Hydroxy-, Nitro-, Amino- oder Alkyl- oder Alkoxygruppen mit jeweils 1 bis 4 Kohlenstoffatomen mono- oder disubstituiert ist, und

$R_3$ Wasserstoff, Halogen, Hydroxy- oder Methoxygruppen darstellt,

sowie deren physiologisch verträgliche Salze.

Für $R_3$ ist die 6- und/oder 7-Stellung bevorzugt.

Gegenstand der Erfindung sind weiterhin Verfahren zur Herstellung sowie pharmazeutische Zubereitungen der Verbindungen der Formel I bzw. deren physiologisch verträglichen Salzen.

Die Verfahren zur Herstellung der Verbindungen der Formel I ist dadurch gekennzeichnet, daß man

a) Verbindungen der Formel II

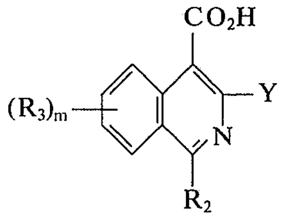

(II)

worin Y gleich Chlor oder Brom ist und $R_2$, $R_3$ und m die zu Anspruch 1 genannte Bedeutung haben mit einem Amin der Formel

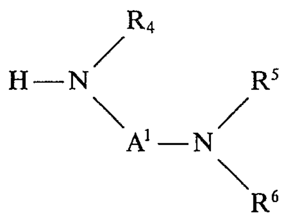

worin $R_4$, $A^1$, $R^5$ und $R^6$ die im Anspruch 1 genannte Bedeutung haben, umsetzt.

b) Verbindungen der Formel III

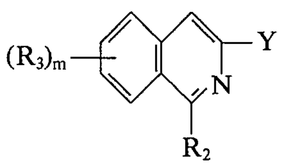

(III)

worin Y gleich Chlor oder Brom ist und $R_2$, $R_3$ und m die obengenannte Bedeutung haben, mit einem Amin der Formel

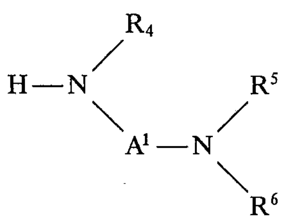

worin $R_4$, $A^1$, $R^5$ und $R^6$ die obengenannte Bedeutung haben, umsetzt.

c) Verbindungen der Formel IV

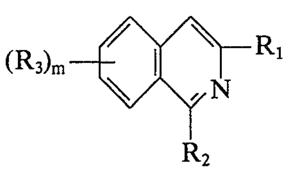

(IV)

**worin $R_1$, $R_2$, $R_3$ und m die oben genannte Bedeutung haben mit der Maßgabe, daß in dem Rest $R_1$ eine sekundäre Aminogruppe vorhanden ist, mit einer entsprechenden Jod-, Chlor- oder Bromverbindung alkyliert oder mit einem Chlorameisensäureester der Formel $Cl-CO_2(C_1-C_3)$-Alkyl, wobei der Alkylrest durch Hydroxy-, Methoxy- oder Äthoxy-Gruppen substituiert sein kann, oder mit 3,4,5-Trimethoxybenzoylchlorid, Methylen-3,4-dioxybenzoylchlorid, 2-Furoylchlorid oder 2-Thienylchlorid umsetzt, oder**

**d) in Verbindungen der Formel I**

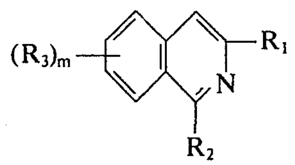

(I)

worin $R_1$, $R_3$ und m die in Anspruch 1 genannte Bedeutung haben, und $R_2$ den Phenylring darstellt, am Phenylring nach üblichen Verfahren die im Anspruch 1 aufgeführten Substituenten einführt.

Bei der Verfahrensweise a) wird mindestens die doppelt äquivalente Menge Amin zugegeben, da ein Mol Amin zur Bildung des abgespaltenen Halogenwasserstoffes gebraucht wird, jedoch ist es manchmal von Vorteil, zur Reaktionsbeschleunigung einen bis zu 15fachen Überschuß des Amins anzuwenden. Arbeitet man mit äquimolaren Mengen Amin, so kann man tertiäre Amine, wie Picolin, 1,4-Diazabicyclo-(2,2,2)-octan, 1,5-Diazabicyclo[5,4,0]undec-5-en oder Kaliumcarbonat als Säurefänger hinzugeben. Als Lösungsmittel kommen, sofern sie zur Umsetzung verwendet werden, indifferente, wasserfreie, organische Lösungsmittel wie Äthylenglykolmonoäthyläther, Octanol, Diäthylenglykoldimethyläther, Diäthylenglykodibutyläther, Toluol, Xylol, Chlorbenzol, Dichlorbenzol, Trichlorbenzol, Dimethylformamid, Dimethylsulfoxid oder Hexamethylphosphorsäuretriamid in Frage. Die Reaktion wird im allgemeinen bei einer Temperatur zwischen 80° und 220°, vorzugsweise zwischen 120° und 180° ausgeführt, und zwar, bis die Kohlendioxydentwicklung beendet ist. Die Ausgangsverbindungen II für das Verfahren a) können gemäß der DE-A-2 811 361 hergestellt werden, z. B. durch Umsetzung von Verbindungen der Formel V

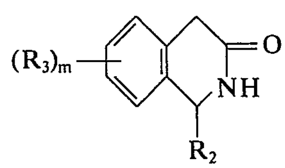

(V)

worin $R_2$, $R_3$ und m die obengenannte Bedeutung haben, mit einem Vilsmeier-Addukt aus einem Säureamid mit einem Säurechlorid oder -bromid zu Verbindungen der Formel VI

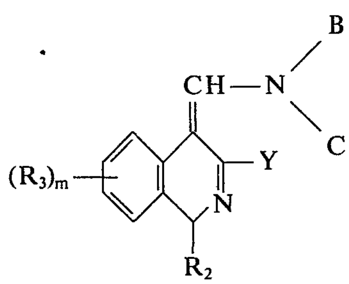

(VI)

worin Y Chlor oder Brom ist und B und C Alkyl oder Cycloalkyl mit einem bis sechs Kohlenstoffatomen oder Phenyl bedeuten, und anschließende Oxydation zu Verbindungen der Formel VII

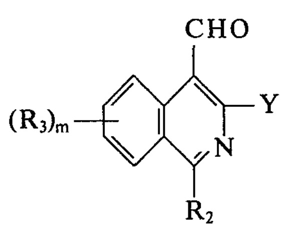

(VII)

worin Y Chlor oder Brom bedeutet und $R_2$, $R_3$ die obengenannte Bedeutung haben. Anschließende Oxydation der Formylgruppe zur Carboxylgruppe liefert dann Verbindungen der Formel II.

Bei dem Verfahren b) werden die Verbindungen der Formel II in der gleichen Weise wie im Verfahren a) mit einem Amin umgesetzt. Die Ausgangsverbindungen III für das Verfahren b) können durch Umsetzung der Verbindungen VIII

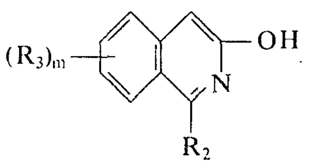

(VIII)

worin $R_2$, $R_3$ und m die obengenannte Bedeutung haben, mit einem Säurechlorid oder -bromid, wie z. B. Thionylchlorid, Phosphoroxychlorid, Phosphortrichlorid, Phosphorpentachlorid, oder Phosphortribromid umsetzt. Die Verbindungen VIII können nach literaturbekannten Vorschriften z. B. durch Cyclisierung von O-Acylphenylessigsäuren mit Ammoniak hergestellt werden (siehe z. B. Khim, Geterotsikl. Soedin., 1976, 8, 1103, und Rocz. chem., 51 [1977], 4, 691).

Nach dem Verfahren c) werden sekundäre Aminogruppen nach an sich bekannten Methoden mit einer entsprechenden Jod-, Chlor- oder Bromverbindung alkyliert.

Nach dem Verfahren d) können in dem aromatischen Rest $R_2$ durch elektrophile Substitution Substituenten eingeführt werden. Dazu gehören vor allem die Halogenierung oder Nitrierung, wobei der Nitrierung besonderes Interesse zukommt. Man geht dabei so vor, daß man Verbindungen der Formel I den üblichen Nitrierbedingungen unterwirft (Schwefelsäure, Salpetersäure, Eiskühlung).

Weiterhin können die am Rest $R_2$ nachträglich eingeführten oder bereits vorhandenen Substituenten $R_3$ zusätzlich, z. B. eine Nitrogruppe durch Reduktion oder eine Methoxygruppe durch Ätherspaltung, verändert werden, so daß weitere Verbindungen der Formel I entstehen. Einige Beispiele aus der Vielzahl der Möglichkeiten sollen dies erläutern. Durch Reduktion einer aromatischen Nitrogruppe erhält man eine Aminoverbindung, z. B., wenn $R_2$ den 4-Nitrophenylrest darstellt, die entsprechende 4-Aminophenylverbindung. Diese Reduktion wird wie üblich durchgeführt, wie z. B. mit Raney-Nickel in Äthanol oder mit Eisenpulver in salzsaurer Lösung.

Die Diazotierung einer aromatischen Aminogruppe mit anschließender Reaktion mit einer nukleophilen Gruppe ist eine weitere Möglichkeit zur Veränderung bereits vorhandener Substituenten. So läßt sich beispielsweise mit salpetriger Säure (üblicherweise aus Natriumnitrit und Schwefelsäure hergestellt) ein Rest $R_2$, wenn er die 4-Aminophenylgruppe bedeutet, bei niedrigen Temperaturen (0—5° C) in das entsprechende Diazoniumsalz überführen, das dann beispielsweise mit Salzsäure in Gegenwart von Kupferchlorid den 4-Chlorphenylrest oder durch Verkochen die 4-Hydroxyphenylgruppe liefert. Die Spaltung einer Alkoxygruppe zur entsprechenden Hydroxyverbindung ist ebenfalls eine Methode zur Umwandlung der verschiedenen Substituenten. So liefert beispielsweise die Ätherspaltung einer 7-Methoxyverbindung ($R_3 = OCH_3$) mit beispielsweise Bromwasserstoff in wäßriger Essigsäure bei Temperaturen zwischen 50 und 120° C die entsprechende 7-Hydroxyverbindung.

Die erfindungsgemäßen Verbindungen haben wertvolle therapeutische Eigenschaften. So zeigen sie neben anderen pharmakologischen Eigenschaften eine Wirkung auf das Zentralnervensystem. Sie können nicht nur die durch den elektrischen Strom ausgelösten Krämpfe verhindern und die Hexabarbitalnarkose verlängern, sondern auch die durch Tetrabenazin verursachte Ptosis bei Mäusen. Auf Grund all dieser Eigenschaften können die erfindungsgemäßen Verbindungen als Wirkstoffe von antidepressiv wirkenden Arzneimitteln angewandt werden.

Die neuen Verbindungen können entweder allein oder mit physiologisch verträglichen Hilfs- oder Trägerstoffen vermischt angewandt werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z. B. Magnesiumcarbonat, Milchzucker oder Maisstärke verwendet werden. Dabei kann die Zubereitung sowohl als Trocken- oder Feuchtgranulat erfolgen. Als ölige Trägerstoffe oder Lösungsmittel kommen beispielsweise besonders pflanzliche und tierische Öle in Betracht, wie z. B. Sonnenblumenöl oder Lebertran.

Eine besondere Anwendungsform liegt in der intravenösen Applikation. Zu diesem Zweck werden die aktiven Verbindungen oder deren physiologisch verträglichen Salze mit den dafür üblichen Substanzen in Lösung gebracht. Solche physiologisch verträglichen Salze werden z. B. mit folgenden Säuren gebildet: Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Zitronensäure, Äpfelsäure, Schleimsäure, Benzoesäure, Salicylsäure, Acetylaminoessigsäure, 4,4'-Methylen-bis(3-hydroxy-2-naphthoesäure) (=Embonsäure), Naphthalin-1,5-disulfonsäure, Ascorbinsäure, Phenylessigsäure, p-Amino-salicylsäure, Hydroxyäthansulfonsäure, Benzolsulfonsäure oder synthetische Harze, die saure Gruppen enthalten, z. B. solche mit Ionenaustauschwirkung. Als Lösungsmittel der entsprechenden physiologisch verträglichen Salze der aktiven Verbindungen für eine intravenöse Applikation kommen z. B. in Frage: Wasser, physiologische Kochsalzlösungen oder Alkohol, wie z. B. Äthanol, Propandiol oder Glycerin, daneben auch Zuckerlösungen, wie z. B. Glukose- oder Mannitlösungen, oder auch eine Mischung aus den verschiedenen genannten Lösungsmitteln.

Beispiel 1
3-N-Methylpiperazino-1-phenyl-isochinolin

30 g 3-Chlor-1-phenyl-isochinolin-4-carbonsäure werden in 90 ml N-Methylpiperazin innerhalb von 2 Stunden auf 150°C erhitzt. Die Reaktionsmischung wird 6 Stunden bei 150°C gehalten, bis die Kohlendioxydentwicklung beendet ist. Die Reaktionsmischung wird abgekühlt, zwischen Wasser und Toluol verteilt, die Toluolphase mit Wasser gewaschen und nach dem Trocknen das Lösungsmittel im Vakuum entfernt. Der ölige Rückstand wird mit äthanolischer Salzsäure in das kristalline Hydrochlorid mit Schmp. 278–282° C überführt.

Das Ausgangsmaterial 3-Chlor-1-phenyl-isochinolin-4-carbonsäure wie wie folgt dargestellt:

53,5 g 3-Chlor-1-phenyl-isochinolin-4-aldehyd wird in 1,5 l Aceton und 500 ml Phosphatpuffer vom pH 7 suspendiert. Bei 40° C trägt man portionsweise im Laufe von 2 Stunden 40 g Kaliumpermanganat ein und rührt bei dieser Temperatur 2 Stunden nach. Das überschüssige Kaliumpermanganat wird mit 10 g Natriumhydrogensulfit zerstört und die Lösung auf 500 ml einrotiert und filtriert. Das Filtrat wird mit konzentrierter Salzsäure auf pH 4 gebracht und mit Essigester gründlich extrahiert. Nach dem Entfernen des Lösungsmittels im Vakuum verbleiben 41,1 g 3-Chlor-1-phenyl-isochinolin-4-carbonsäure mit Schmp. 208° C.

Wie vorstehend werden die 3-substituierten Isochinoline der Beispiele 2–8 (Tabelle 1) aus den 3-Chlor-1-phenyl-isochinolin-4-carbonsäuren und den entsprechenden Basen dargestellt.

Tabelle 1

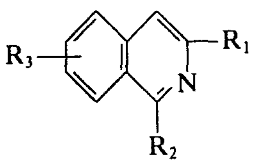

| Beispiel | $R_1$ | $R_2$ | $R_3$ | Schmp.°C/Salz (Schmp.°C) |
|---|---|---|---|---|
| 2 | 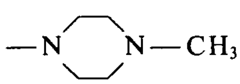 | $—C_6H_5$ | 7-Cl | 131–133/HCl (307–309) |
| 3 | 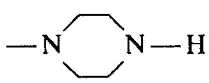 | $—C_6H_5$ | H | 107–118/HCl (284–287) |
| 4 | 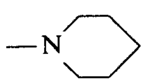 | $—C_6H_5$ | H | Öl/HCl (115–157) |
| 5 | 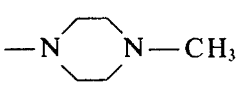 | $4\text{-Cl}—C_6H_4$ | H | /HCl (277–280) |
| 6 | 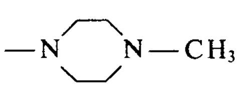 | $2\text{-}CH_3—C_6H_4$ | H | Harz/HCl (216–218) |
| 7 | 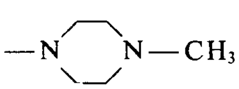 | $2\text{-F}—C_6H_4$ | H | Harz/HCl (146–150) |
| 8 | 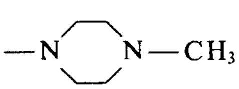 | $4\text{-}NO_2—C_6H_4$ | H | /HCl (287–289) |

Beispiel 9
3-N-Butyl-piperazino-1-phenyl-isochinolin

6,65 g 3-Piperazino-1-phenyl-isochinolin und 4,73 g N-Butylbromid werden mit 4,88 g Natriumcarbonat und 0,2 g Kaliumjodid in 150 ml Toluol 4 Tage am Rückfluß gekocht. Das Reaktionsgemisch wird nach dem Abkühlen mit Wasser gewaschen, getrocknet und einrotiert. Es verbleibt ein braunes Öl, das mit äthanolischer Salzsäure 5,7 g kristallines Hydrochlorid des 3-N-Butylpiperazino-1-phenyl-isochinolin mit Schmp. 216–218° C ergibt.

Beispiel 10
3-N-Methylpiperazino-1-(4-aminophenyl)-isochinolin

4,8 g 3-N-Methyl-piperazino-1-(4-nitrophenyl)-isochinolin-Hydrochlorid werden in 900 ml Methanol mit 1 g Palladium auf Tierkohle (10%ig) bei Raumtemperatur und einer Atmosphäre Wasserstoffdruck hydriert. Nach 1 Stunde ist die theoretische Wasserstoffmenge aufgenommen; der Katalysator wird abfiltriert und die Lösung einrotiert. Man isoliert 3,9 g 3-N-Methylpiperazino-1-(4-aminophenyl)-isochinolin-Hydrochlorid mit Schmp. 247—248° C.

**Patentansprüche**

1. Isochinoline der Formel I

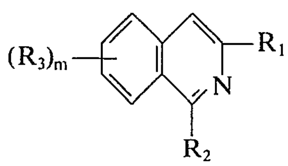

(I)

worin
m eins oder zwei bedeutet,
$R_1$ entweder einen über das Ringstickstoffatom gebundenen Pyrrolidino-, Piperidino-, Hexamethylenimino-, Morpholino-, 4-Hydroxypiperidino-, 4-Carbäthoxypiperidino- oder den 1-Piperazinylrest

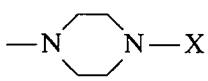

darstellt, worin X Wasserstoff, $C_1-C_4$-Alkyl, $\beta$-Hydroxyäthyl, Methylen-3,4-dioxybenzyl, Phenyl, durch Methoxy, Chlor, Nitro oder Amino substituiertes Phenyl, 3,4,5-Trimethoxybenzoyl, Methylen-3,4-dioxybenzoyl, 2-Furoyl, 2-Thienyl, $C_1-C_3$-Alkoxycarbonyl bedeutet, wobei der $C_1-C_3$-Alkylrest im letzteren durch OH, Methoxy oder Äthoxy substituiert sein kann, oder ein Aminorest der Formel

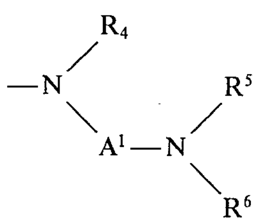

ist, in dem $R_4$ Wasserstoff oder $C_1-C_4$-Alkyl, $A_1$ eine geradkettige oder verzweigte $C_2-C_6$-Alkylengruppe, die durch Hydroxy- oder $C_1-C_4$-Alkoxygruppen substituiert sein kann, sowie $R_5$ und $R_6$ gleich oder verschieden sind und Wasserstoff oder einen geradkettigen oder verzweigten Alkylrest mit 1 bis 6 Kohlenstoffatomen bedeuten, oder zusammen mit dem Stickstoffatom einen heterocyclischen Ring mit bis zu 7 Kohlenstoffatomen darstellen,
$R_2$ einen Phenylrest bedeutet, der gegebenenfalls mit Halogen, Hydroxy-, Nitro-, Amino- oder Alkyl- oder Alkoxygruppen mit jeweils 1 bis 4 Kohlenstoffatomen mono- oder disubstituiert ist, und
$R_3$ Wasserstoff, Halogen, Hydroxy- oder Methoxygruppen darstellt,
sowie deren physiologisch verträgliche Salze.

2. Isochinoline nach Anspruch 1, dadurch gekennzeichnet, daß sich ein Substituent $R_3$ in 6- und/oder 7-Stellung befindet.

3. Verfahren zur Herstellung der Verbindungen gemäß Anspruch 1, dadurch gekennzeichnet, daß man
a) Verbindungen der Formel II

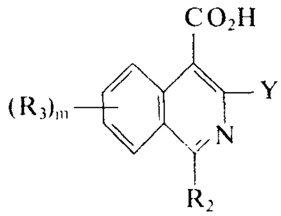

(II)

worin Y gleich Chlor oder Brom ist und $R_2$, $R_3$ und m die im Anspruch 1 genannte Bedeutung haben, mit einem Amin der Formel

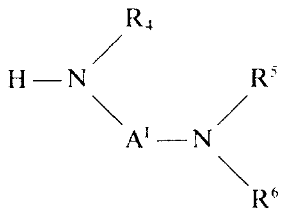

worin $R_4$, $A^1$, $R^5$ und $R^6$ die im Anspruch 1 genannte Bedeutung haben, umsetzt.

b) Verbindungen der Formel III

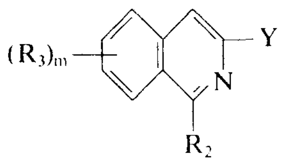

(III)

worin Y gleich Chlor oder Brom ist und $R_2$, $R_3$ und m die im Anspruch 1 genannte Bedeutung haben, mit einem Amin der Formel

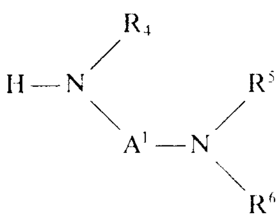

worin $R_4$, $A^1$, $R^5$ und $R^6$ die im Anspruch 1 genannte Bedeutung haben, umsetzt.

c) Verbindungen der Formel IV

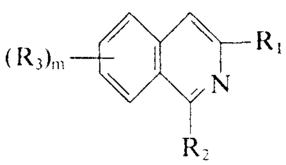

(IV)

worin $R_1$, $R_2$, $R_3$ und m die in Anspruch 1 genannte Bedeutung haben mit der Maßgabe, daß in dem Rest $R_1$ eine sekundäre Aminogruppe vorhanden ist, mit einer entsprechenden Jod-, Chlor- oder Bromverbindung alkyliert oder mit einem Chlorameisensäureester der Formel $Cl-CO_2(C_1-C_3)$-Alkyl, wobei der Alkylrest durch Hydroxy-, Methoxy- oder Äthoxy-Gruppen substituiert sein kann, oder mit 3,4,5-Trimethoxybenzoylchlorid, Methylen-3,4-dioxybenzoylchlorid, 2-Furoylchlorid oder 2-Thienylchlorid umsetzt, oder

d) in Verbindungen der Formel I

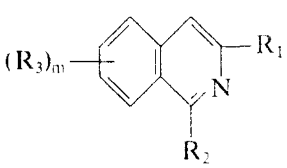

(I)

worin $R_1$, $R_3$ und m die in Anspruch 1 genannte Bedeutung haben und $R_2$ den Phenylring darstellt, am Phenylring nach üblichen Verfahren die in Anspruch 1 aufgeführten Substituenten einführt.

4. Pharmazeutische Zubereitung, dadurch gekennzeichnet, daß sie eine Verbindung gemäß Anspruch 1 enthält oder aus dieser besteht.

5. Verfahren zur Herstellung einer pharmazeutischen Zubereitung nach Anspruch 4, dadurch gekennzeichnet, daß man eine Verbindung gemäß Anspruch 1, gegebenenfalls zusammen mit üblichen pharmazeutischen Trägern und/oder Stabilisatoren, in eine für pharmazeutische Zwecke geeignete Darreichungsform bringt.

**Claims**

1. Isoquinolines of the formula I

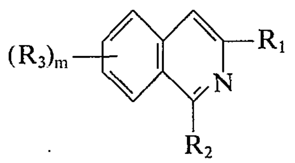

(I)

in which

m is 1 or 2,

$R_1$ is either — bound via the nitrogen atom — a pyrrolidino, piperidino, hexamethyleneimino, morpholino, 4-hydroxypiperidino, 4-carbethoxypiperidino or 1-piperazinyl

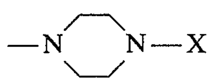

in which in turn X is hydrogen, $C_1-C_4$-alkyl, $\beta$-hydroxyethyl, methylene-3,4-dioxybenzyl, phenyl, phenyl being substituted by methoxy, chloro, nitro or amino, 3,4,5-trimethoxybenzoyl, methylene-3,4-dioxybenzoyl, 2-furoyl, 2-thienyl, $C_1-C_3$-alkoxycarbonyl, in which in turn the $C_1-C_3$-alkyl may be substituted by hydroxy, methoxy or ethoxy; or $R_1$ is an amino moiety of the formula

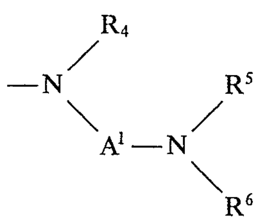

in which in turn $R_4$ is hydrogen or $C_1-C_4$-alkyl, $A^1$ is straight chain or branched $C_2-C_6$-alkylene, which in turn may be substituted by hydroxy or $C_1-C_4$-alkoxy, $R_5$ und $R_6$ are the same or different and represent hydrogen, or straight chain or branched $C_1-C_6$-alkyl, or $R_5$ and $R_6$ represent together with the nitrogen atom to which they are attached a heterocyclus having up to 7 carbon atoms,

$R_2$ is phenyl which may be mono- or di-substituted by halogen, hydroxy, nitro, amino, alkyl or alkoxy each having 1 to 4 carbon atoms, and

$R_3$ is hydrogen, halogen, hydroxy or methoxy;

and the salts of such compounds with a physiologically compatible acid.

2. Isoquinolines according to Claim 1, wherein a substituent $R_3$ is in 6- and/or 7-position.

3. A process for preparing the compounds according to Claim 1, wherein

a) compounds of the formula II

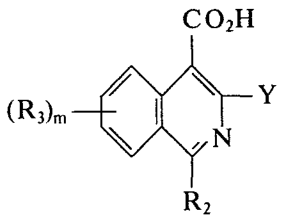

(II)

in which Y is chlorine or bromine and $R_2$, $R_3$ and m are defined as in Claim 1, are reacted with an amine of the formula

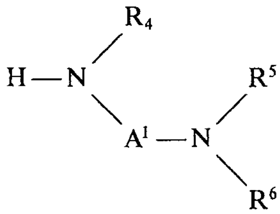

in which $R_4$, $A^1$, $R^5$ and $R^6$ are defined as in Claim 1,

b) compounds of the formula III

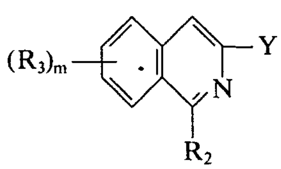

(III)

in which Y is chlorine or bromine and $R_2$, $R_3$ and m are defined as in Claim 1, are reacted with an amine of the formula

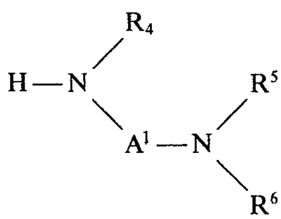

in which $R_4$, $A^1$, $R^5$ and $R^6$ are defined as in Claim 1,

c) compounds of the formula IV

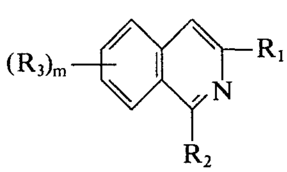

(IV)

in which $R_1$, $R_2$, $R_3$ and m are defined as in Claim 1 with the proviso that in the radical $R_1$ a secondary amino group is present, are alkylated with a corresponding iodine, chlorine or bromine compound; or with a chloroformic acid ester of the formula $Cl-CO_2(C_1-C_3)$-alkyl, the alkyl radicals carrying optionally hydroxy, methoxy or ethoxy groups; or with 3,4,5-trimethoxy-benzoylchlorid, methylene-3,4-dioxybenzoylchlorid, 2-furoylchlorid or 2-thienylchlorid; or

d) introducing in the phenyl ring in compounds of the formula I

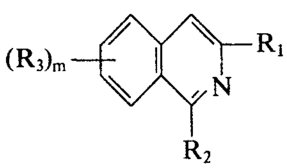

(I)

in which $R_1$, $R_3$ and m are as defined in Claim 1 and $R_2$ represents the phenyl ring, the substituents cited in Claim 1, according to common methods.

4. Pharmaceutical compositions completely or partially consisting of a compound of the formula I.

5. Process for the preparation of a pharmaceutical composition as claimed in Claim 4, which comprises bringing a compound of the formula I, optionally in conjunction with the common pharmaceutical carriers and/or stabilizers, into a dosage unit form which is suitable for pharmaceutical purposes.

**Revendications**

1. Isoquinoléines répondant à la formule I

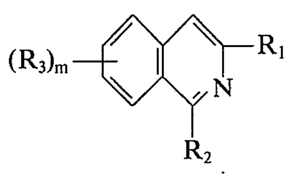

(I)

dans laquelle

m est égal à 1 ou 2,

$R_1$ désigne soit un radical pyrrolidino, pipéridino, hexaméthylènimino, morpholino, 4-hydroxypipéridino, 4-carbéthoxypipéridino lié par l'atome d'azote du noyau, ou le radical 1-pipérazinyle,

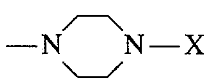

où X est l'hydrogène, un groupe alcoyle en $C_1$ à $C_4$ $\beta$-hydroxyéthyle, méthylène-3,4-dioxybenzyle, phényle, phényle substitué par un radical méthoxy, chloro, nitro ou amino, 3,4,5-triméthoxy benzoyle, méthylène-3,4-dioxybenzoyle, 2-furoyle, 2-thiényle, alcoxy ($C_1$ à $C_3$) carbonyle, le radical alcoyle en $C_1$ à $C_3$ pouvant être substitué dans ce dernier par un groupe OH, méthoxy ou éthoxy, soit un radical amino répondant à la formule

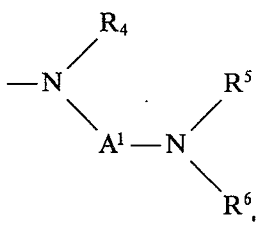

dans laquelle $R_4$ représente l'hydrogène ou un groupe alcoyle en $C_1$ á $C_4$, $A_1$ désigne un groupe alcoylène en $C_2$ à $C_6$ linéaire ou ramifiée, qui peut être substitué par des groupes hydroxy ou alcoxy en $C_1$ à $C_4$, $R_5$ et $R_6$ sont identiques ou différents et désignent l'hydrogène ou un radical alcoyle linéaire ou ramifié en $C_1$ à $C_6$, ou représentent avec l'atome d'azote un noyau hétérocyclique ayant jusqu'à 7 atomes de carbone,

$R_2$ désigne un radical phényle qui peut éventuellement être mono- ou disubstitué par des groupes halogène, hydroxy, nitro, amino ou des groupes alcoyle ou alcoxy en $C_1$ à $C_4$, et

$R_3$ désigne l'hydrogène, un halogène, des groupes hydroxy ou méthoxy,

ainsi que leurs sels physiologiquement acceptables.

2. Isoquinoléines suivant la revendication 1, caractérisées en ce qu'un substituant $R_3$ se trouve en position 6 et/ou en position 7.

3. Procédé de préparation des composés suivant la revendication 1, caractérisé en ce que

a) on fait réagir des composés répondant à la formule II

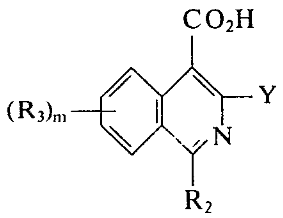

(II)

dans laquelle Y est le chlore ou le brome et $R_2$, $R_3$ et m ont la signification indiquée dans la revendication 1, avec une amine répondant à la formule

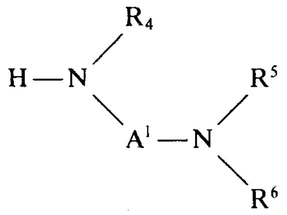

dans laquelle $R_4$, $A_1$, $R_5$ et $R_6$ ont la signification indiquée dans la revendication 1, et

b) on fait réagir des composés répondant à la formule III

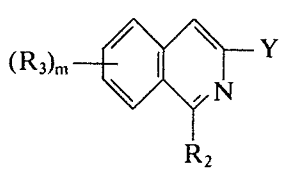

(III)

dans laquelle Y est le chlore ou le brome et $R_2$, $R_3$ et m ont la signification indiquée dans la revendication 1, avec une amine répondant à la formule

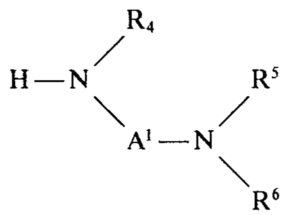

dans laquelle $R_4$, $A^1$, $R^5$ et $R^6$ ont la signification indiquée dans la revendication 1,

c) on alcoyle des composés répondant à la formule

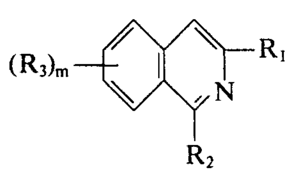

(IV)

**dans laquelle $R_1$, $R_2$ $R_3$ et m ont la signification indiquée dans la revendication 1, sous réserve que dans le radical $R_1$, un groupe amino secondaire soit présent, avec un composé de l'iode, du chlore ou du brome correspondant, ou on les fait réagir avec un ester chloroformique répondant à la formule $Cl-CO_2$-alcoyle($C_1$ à $C_3$), le radical alcoyle pouvant être substitué par des groupes hydroxy, méthoxy ou éthoxy, ou on les fait réagir avec le chlorure de 3,4,5-triméthoxybenzoyle, le chlorure de méthylène-3,4-dioxybenzoyle, le chlorure de 2-furoyle ou le chlorure de 2-thiényle, ou**

**d) dans des composés répondant à la formule I**

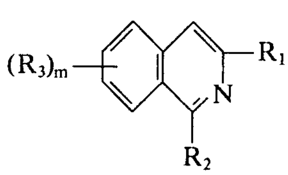

(I)

dans laquelle $R_1$, $R_3$ et m ont la signification indiquée dans la revendication 1, et $R_2$ représente le noyau phényle, on introduit par les procédés habituels les substituants indiqués dans la revendication 1.

4. Préparation pharmaceutique, caractérisée en ce qu'elle contient un composé suivant la revendication 1 ou est constituée d'un tel composé.

5. Procédé de préparation d'une préparation pharmaceutique, suivant la revendication 4, caractérisé en ce qu'on présente un composé suivant la revendication 1, associé le cas échéant à des supports et/ou stabilisants pharmaceutiques habituels, sous une forme d'administration convenant à des fins thérapeutiques.